# FASCICULE DE BREVET EUROPEEN

(11) **EP 1 867 304 B1**
(45) Date de publication et mention de la délivrance du brevet: **04.08.2010**
(21) Numéro de dépôt: 06012260.3
(22) Date de dépôt: 14.06.2006
(51) Int. Cl.: A61F 2/44, A61F 2/30

(54) **Dispositif de remplacement vertébral**
Vorrichtung zum Wirbelkörperersatz
Device for replacing vertebrae

(43) Date de publication de la demande: 19.12.2007
(73) Titulaire: Eden Spine Europe SA, 1207 Genève (CH)
(72) Inventeur: Rogeau, Dominique, 08034 Barcelone (ES); Ben-Mokhtar, Mourad, 1207 Geneve (CH)
(74) Mandataire: Micheli & Cie SA

(56) Documents cités:
- EP-A1- 0 188 954
- WO-A-92/01428
- WO-A-98/46173
- WO-A-03/013399
- WO-A2-99/63913
- FR-A1- 2 762 778
- FR-A1- 2 850 563
- US-A1- 2003 045 877

## Description

La présente invention concerne un dispositif de remplacement d'une ou plusieurs vertèbres, conçu pour maintenir un espacement entre deux vertèbres.

On connaît par le document FR 2 850 563 un tel dispositif comprenant une pièce mâle montée dans une pièce femelle, un patin d'ancrage guidé en translation et bloqué en rotation sur la pièce mâle, une surface d'ancrage sur la pièce femelle et un organe de commande transformant un mouvement de rotation en un mouvement de translation du patin d'ancrage pour amener le patin dans une première position où les pièces mâle et femelle sont bloquées l'une par rapport à l'autre ou dans une seconde position où les pièces femelle et mâle peuvent coulisser axialement librement l'une par rapport à l'autre.

Ce dispositif présente l'inconvénient que la pièce mâle n'est bloquée axialement par rapport à la pièce femelle que d'un côté, ce qui, à la longue, peut être préjudiciable à la qualité de la liaison entre les pièces mâle et femelle et donc à la sécurité d'utilisation du dispositif.

On connaît également par le document FR 2 730 158 un dispositif de remplacement vertébral comprenant une pièce mâle à paroi élastique et une pièce femelle. Les pièces mâle et femelle comprennent des crantages complémentaires en forme de dents de scie asymétriques. Ces crantages asymétriques permettent un déplacement en distraction des pièces mâle et femelle, par glissement du crantage de la pièce mâle sur le crantage de la pièce femelle et déformation élastique de la paroi de la pièce mâle, et bloquent les pièces mâle et femelle en compression.

L'avantage d'un tel dispositif est que le réglage de la distance entre les pièces mâle et femelle est simple à effectuer, puisqu'il ne nécessite que de distraire les pièces mâle et femelle jusqu'à la position souhaitée, ce mouvement de distraction étant irréversible. Toutefois, l'élasticité conférée à la pièce mâle peut, sur la durée, fragiliser le dispositif et donc nuire à sa sécurité d'utilisation. De plus, du fait que l'enveloppe externe de la pièce mâle coopère directement avec l'enveloppe interne de la pièce femelle, les diamètres de ces enveloppes doivent être très précis, ce qui complique la fabrication.

Le document WO 98/46173 décrit un dispositif de remplacement vertébral comprenant une pièce mâle engagée dans une pièce femelle. La paroi périphérique de la pièce femelle comprend un logement qui reçoit un ressort en forme d'anneau fendu. La face interne du ressort définit un crantage qui coopère avec la surface périphérique, elle aussi crantée, de la pièce mâle pour bloquer axialement les pièces mâle et femelle l'une par rapport à l'autre. Le dispositif peut être équipé d'une vis de verrouillage traversant radialement la paroi périphérique de la pièce femelle et débouchant dans le logement précité.

La présente invention vise à proposer un dispositif de remplacement vertébral qui, notamment, soit capable d'offrir une grande sécurité d'utilisation. A cette fin il est prévu un dispositif de remplacement vertébral tel que défini dans la revendication 1 annexée. Des modes de réalisation particuliers sont définis dans les revendications dépendantes 2 à 11.

D'autres caractéristiques et avantages de la présente invention apparaîtront à la lecture de la description détaillée suivante faite en référence aux dessins annexés dans lesquels :
- les figures 1 à 3 montrent respectivement en perspective et selon deux coupes longitudinales prises dans deux plans perpendiculaires un dispositif de remplacement vertébral selon l'invention ;
- la figure 4 montre en perspective une pièce définissant des lames ressort utilisée dans le dispositif selon l'invention ;
- la figure 5 montre en perspective un patin utilisé dans le dispositif selon l'invention ;
- la figure 6 montre en coupe longitudinale le dispositif de remplacement vertébral selon l'invention, dans un état où des pièces mâle et femelle de ce dispositif peuvent coulisser librement l'une par rapport à l'autre ;
- les figures 7 et 8 montrent respectivement en perspective et en coupe longitudinale le dispositif selon l'invention avec un plateau supérieur dans une position réglée inclinée ;
- la figure 9 montre en coupe longitudinale un autre dispositif de remplacement vertébral ne faisant pas partie de l'objet revendiqué ;
- la figure 10 montre en coupe longitudinale le dispositif de remplacement de la figure 9, dans un état où une vis de verrouillage est desserrée pour permettre le réglage de l'espacement entre des pièces mâle et femelle de ce dispositif ;
- les figures 11 à 13 montrent respectivement en perspective vue de deux côtés différents et en coupe longitudinale un autre dispositif de remplacement vertébral, ne faisant pas partie de l'objet revendiqué ;
- les figures 14 et 15 montrent en perspective deux pièces utilisées dans le dispositif des figures 11 à 13 ; et
- la figure 16 montre en coupe longitudinale le dispositif des figures 11 à 13, dans un état où une vis de verrouillage est desserrée pour permettre le réglage de l'espacement entre deux pièces de ce dispositif.

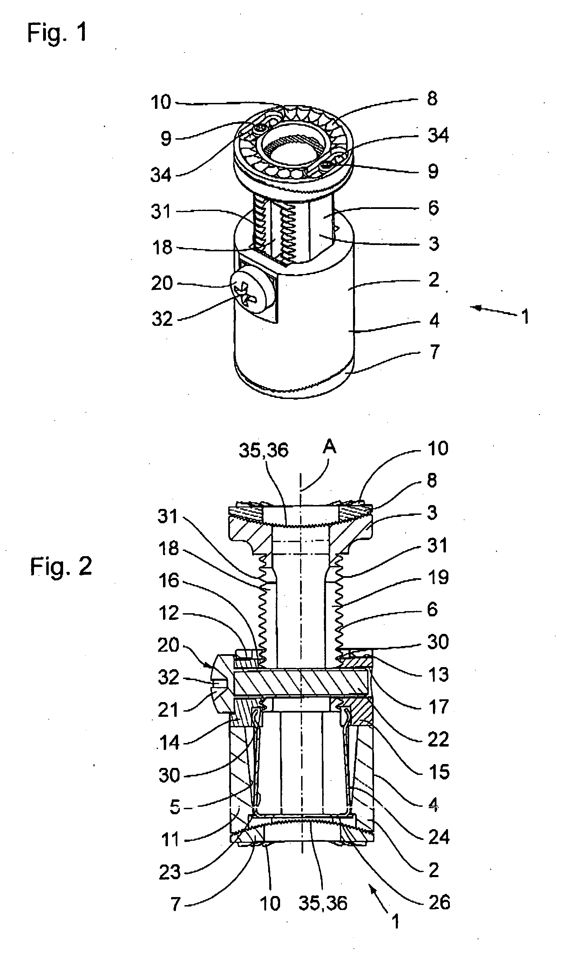

En référence aux figures 1 à 3, un dispositif ou implant de remplacement vertébral 1 selon l'invention comprend une pièce femelle 2 et une pièce mâle 3 engagée partiellement dans la pièce femelle 2. Les pièces femelle et mâle 2, 3 sont des pièces allongées et creuses, ayant un axe longitudinal commun A. La surface périphérique externe 4 de la pièce femelle 2 est à section circulaire. La surface périphérique interne 5 de la pièce femelle 2 et la surface périphérique externe 6 de la pièce mâle 3 ont une section carrée ou rectangulaire à coins arrondis et sont adaptées l'une à l'autre pour permettre un coulissement axial des pièces 2, 3 l'une par rapport à l'autre sans ou avec peu de jeu radial. Des plateaux dits inférieur 7 et supérieur 8 sont fixés respectivement aux pièces femelle et mâle 2, 3 par des vis 9 et comportent des éléments d'ancrage 10 destinés à ancrer le dispositif 1 dans les vertèbres sus- et sous-jacentes.

La pièce femelle 2 comporte dans sa paroi périphérique 11 deux logements diamétralement opposés 12, 13 dans lesquels deux patins d'ancrage 14, 15 sont guidés en translation radiale, c'est-à-dire dans une direction orthogonale à l'axe A. Les logements 12, 13 et les patins 14, 15 ont des formes carrées ou rectangulaires complémentaires, empêchant les patins 14, 15 de tourner dans les logements 12, 13. Les patins 14, 15 comportent chacun un alésage traversant 16, 17 situé en regard d'une lumière oblongue respective 18, 19 pratiquée dans la paroi périphérique de la pièce mâle 3. Les alésages 16, 17 et les lumières 18, 19 permettent le passage d'une vis de commande 20 à travers les patins 14, 15 et la pièce mâle 3.

La vis 20 comporte une tête 21 et une tige 22. L'extrémité de la tige 22 la plus éloignée de la tête 21 est filetée et coopère avec un taraudage que comporte l'alésage 17 du patin 15. L'autre extrémité de la tige 22, proche de la tête 21, ne comporte elle pas de filetage. Le patin 14 est monté librement autour de cette autre extrémité.

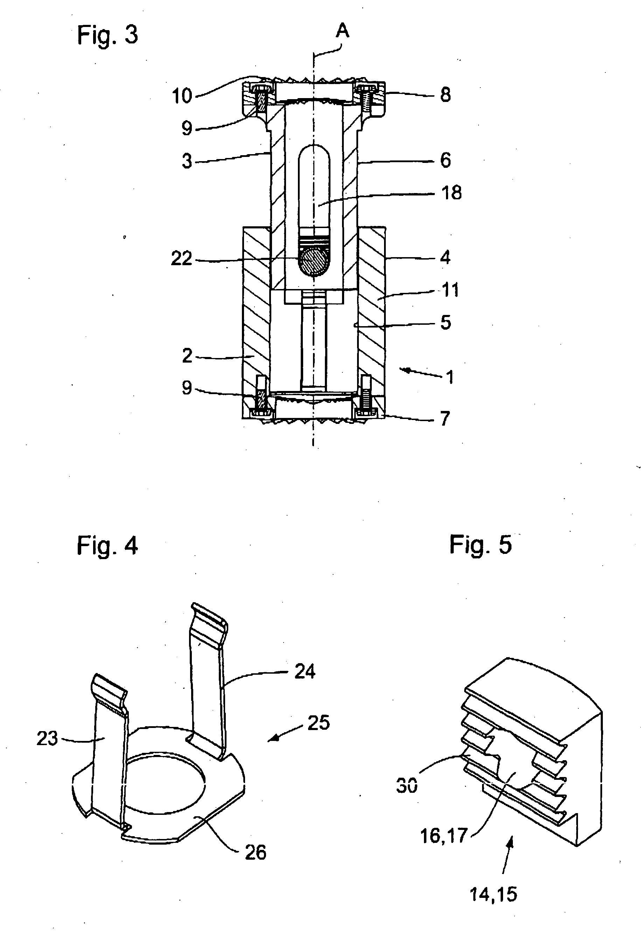

Les patins 14, 15 sont soumis à une action élastique qui tend à les écarter de la pièce mâle 3. Cette action élastique est exercée par deux lames ressorts 23, 24 identiques agissant respectivement sur les patins 14, 15. Les lames ressorts 23, 24 font partie d'une même pièce monobloc 25, montrée à la figure 4, pièce qui comporte une base rigide 26 qui repose sur le fond de la pièce femelle 2 et depuis laquelle s'étendent les lames 23, 24 sensiblement parallèlement à l'axe A.

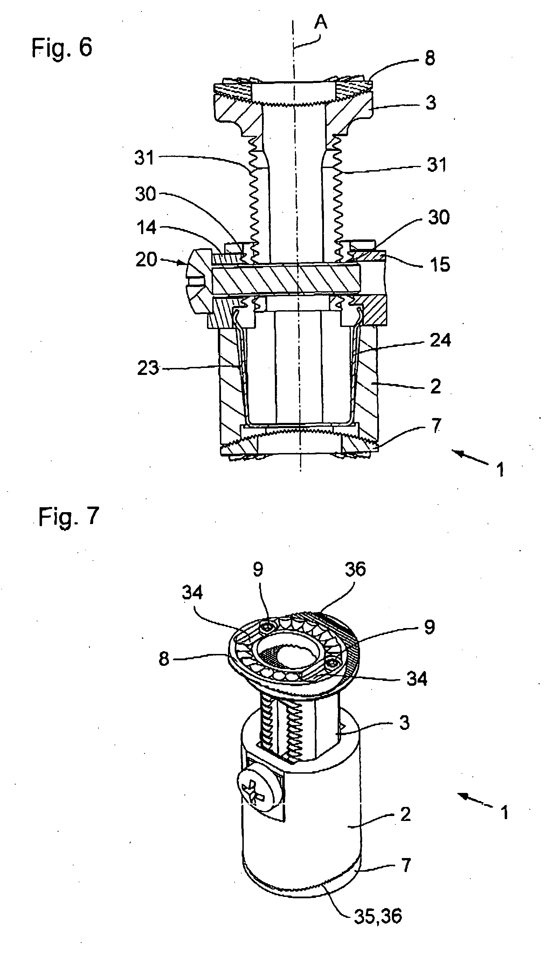

Comme montré aux figures 1, 2 et 5, les surfaces mutuellement en regard des patins 14, 15 et de la pièce mâle 3 comportent des crantages complémentaires en dents de scie 30, 31. Ces crantages 30, 31 coopèrent entre eux lorsque les patins 14, 15 sont dans une première position (figure 1, 2), ancrée dans la surface périphérique externe 6 de la pièce mâle 3, et ne coopèrent plus entre eux lorsque les patins 14, 15 sont dans une seconde position, éloignée de la pièce mâle 3 (figure 6). Dans leur première position, les patins 14, 15 bloquent complètement les pièces femelle et mâle 2, 3 l'une par rapport à l'autre. Dans la seconde position des patins 14, 15, les pièces femelle et mâle 2, 3 sont libres de coulisser l'une par rapport à l'autre suivant l'axe A, permettant au chirurgien de régler l'espacement entre les plateaux 7, 8, dans des limites définies par les lumières oblongues 18, 19 contre la paroi desquelles peut buter la tige 22 de la vis 20. Une rotation des pièces femelle et mâle 2, 3 l'une par rapport à l'autre autour de l'axe A est empêchée ou limitée par les lumières oblongues 18, 19 qui ont une largeur égale ou légèrement supérieure au diamètre de la tige 22.

La translation des patins 14, 15 de l'une à l'autre de leurs première et seconde positions est commandée par une rotation de la vis de commande 20 autour de son axe, rotation qui peut être imprimée au moyen d'un tournevis reçu dans une empreinte correspondante 32 de la tête 21. Une rotation de la vis de commande 20 dans un sens rapproche les patins 14, 15 de la pièce mâle 3 et les amène donc dans la première position, tandis qu'une rotation de la vis de commande 20 dans l'autre sens éloigne les patins 14, 15 de la pièce mâle 3 et les amène donc dans la seconde position. Plus précisément, lorsque la vis 20 est tournée dans le premier sens, le patin 14 est poussé par la tête 21 jusqu'à ce qu'il arrive en butée contre la pièce mâle 3 tandis que l'autre patin 15, commandé par la vis 20 à la manière d'un écrou bloqué en rotation, se rapproche de la pièce mâle 3 jusqu'à venir en butée contre elle, ces mouvements des patins 14, 15 s'effectuant à l'encontre de l'action élastique exercée par les lames ressort 23, 24. Lorsque la vis 20 est tournée dans le second sens, les patins 14, 15 sont écartés de la pièce mâle 3 sous l'action des ressorts 23, 24.

On appréciera que le verrouillage en position des pièces femelle et mâle 2, 3, obtenu lorsque la vis 20 est complètement serrée, est particulièrement fiable puisqu'il est assuré en deux points diamétralement opposés de la pièce mâle 3. Ce verrouillage s'effectue de plus simplement, par la rotation d'une seule vis de commande 20 accessible depuis un côté du dispositif.

Dans une variante de réalisation (non représentée), l'extrémité de la tige 22 de la vis 20 proche de la tête 21 comporte un filetage dont le pas est inversé par rapport à celui de l'autre extrémité de la tige 22. Ce filetage coopère avec un taraudage correspondant du patin 14.

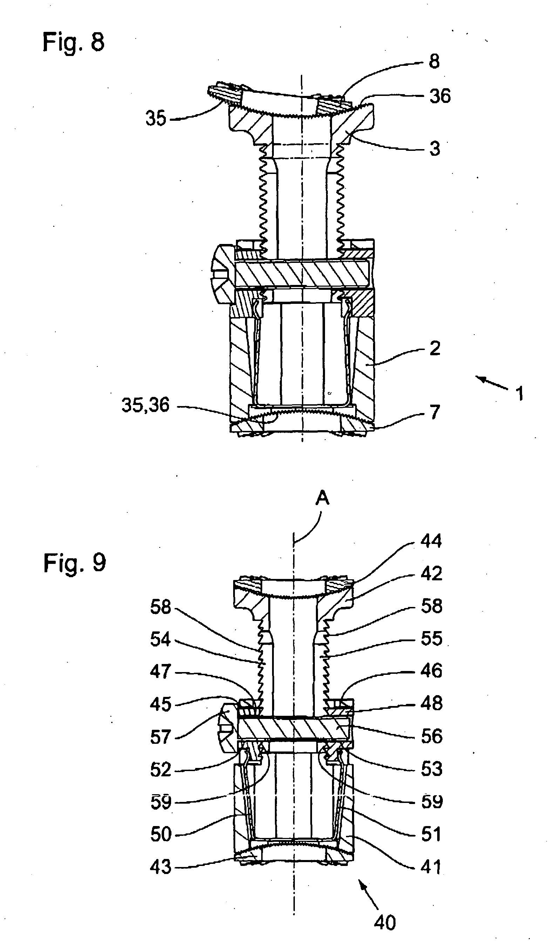

Selon une autre caractéristique de l'invention, l'inclinaison des plateaux 7, 8 par rapport à l'axe A des pièces femelle et mâle 2, 3 est réglable. Comme on peut le voir sur les figures 1 et 7, les vis 9 qui servent à fixer les plateaux 7, 8 sur la face inférieure de la pièce femelle 2 et la face supérieure de la pièce mâle 3, respectivement, coopèrent avec des ouvertures traversantes oblongues 34 des plateaux 7, 8. De plus, la face supérieure du plateau inférieur 7 et la face inférieure de la pièce femelle 2 ont des crantages complémentaires 35, 36 et un profil en arc de cercle, respectivement convexe et concave, de même centre et de même rayon. Ces faces sont maintenues l'une contre l'autre par les vis 9, les crantages 35, 36 coopérant entre eux pour définir et maintenir l'inclinaison du plateau 7. La face inférieure du plateau supérieur 8 et la face supérieure de la pièce mâle 3 ont elles aussi des crantages complémentaires 35, 36 et un profil en arc de cercle, respectivement convexe et concave, de même centre et de même rayon. Ces faces sont maintenues l'une contre l'autre par les vis 9, les crantages 35, 36 coopérant entre eux pour définir et maintenir l'inclinaison du plateau 8, comme montré sur les figures 7 et 8. Les crantages 35, 36 ont un profil en dents de scie et s'étendent dans une direction orthogonale à celle suivant laquelle les ouvertures oblongues 34 sont orientées. Pour modifier l'inclinaison des plateaux 7, 8 (cf. figures 7, 8), le chirurgien dévisse partiellement les plateaux 7, 8 pour désolidariser les crantages 35, 36, déplace les plateaux 7, 8 par rapport aux pièces femelle et mâle 2, 3 dans la direction des ouvertures oblongues 34 et les positionne de manière appropriée au moyen des crantages 35, 36, puis revisse les plateaux 7, 8.

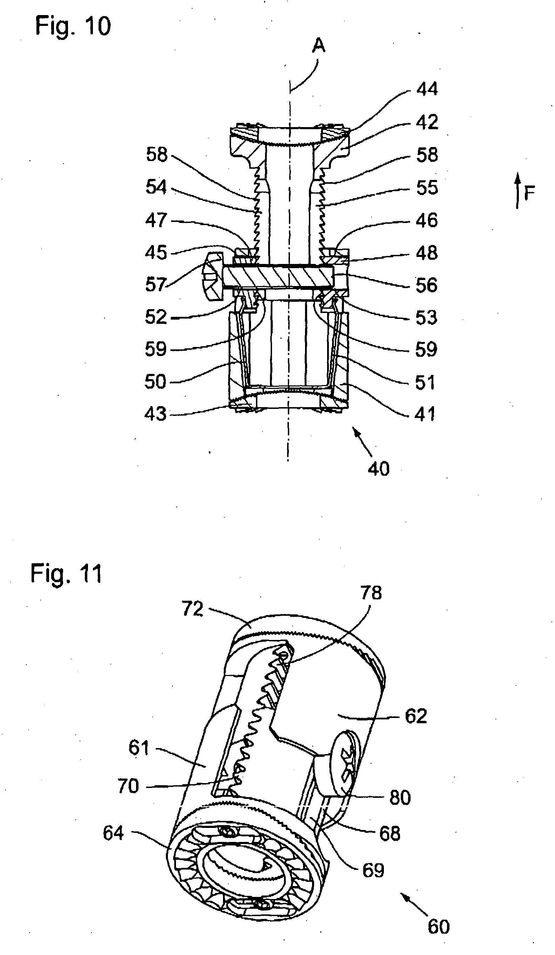

La figure 9 montre un autre dispositif de remplacement vertébral 40. Le dispositif 40 comporte des pièces femelle et mâle 41, 42 de même forme que les pièces 2, 3 du dispositif 1 est auxquelles sont fixés des plateaux inférieur et supérieur 43, 44 d'inclinaison réglable identiques aux plateaux 7, 8. Comme dans le dispositif 1, la paroi périphérique de la pièce femelle 41 comporte deux logements diamétralement opposés 45, 46 qui reçoivent des patins crantés 47, 48 guidés en translation radiale mais bloqués en rotation dans les logements 45, 46. Les patins 47, 48 sont soumis à l'action de deux lames ressort 50, 51 faisant partie d'une même pièce monobloc posée sur le fond de la pièce femelle 41. Des alésages traversants 52, 53 pratiqués dans les patins 47, 48 et des lumières oblongues 54, 55 pratiquées dans la paroi périphérique de la pièce mâle 42 et identiques aux lumières 18, 19 du dispositif 1 permettent le passage de la tige 56 d'une vis, tige 56 dont l'extrémité la plus éloignée de la tête de vis 57 est filetée et coopère avec un taraudage de l'alésage 53 du patin 48 et dont l'extrémité la plus proche de la tête 57, autour de laquelle est situé le patin 47, est sans filetage, permettant à la vis 56, 57 de tourner librement par rapport au patin 47. Ce dispositif 40 diffère du dispositif 1 en ce que :
- les lames ressort 50, 51 exercent sur les patins 47, 48 une action élastique qui maintient en permanence ceux-ci en contact avec les crantages en dents de scie, référencés 58, de la pièce mâle 42,
- la vis 56, 57 exerce une simple fonction de verrouillage, et non pas une fonction de commande de la position des patins 47, 48,
- les crantages 58 de la pièce mâle 42 et les crantages complémentaires, référencés 59, des patins 47, 48 sont asymétriques, pour permettre, lorsque la vis 56, 57 est suffisamment desserrée (figure 10), un coulissement en distraction de la pièce mâle 42 par rapport à la pièce femelle 41, c'est-à-dire dans le sens de la flèche F de la figure 10, par glissement des crantages 58 de la pièce mâle 42 sur ceux, 59, des patins 47, 48 à l'encontre de l'action des lames ressort 50, 51, et pour bloquer les pièces 41, 42 en compression, c'est-à-dire empêcher un coulissement de la pièce mâle 42 par rapport à la pièce femelle 41 dans le sens contraire de la flèche F, même lorsque la vis 56, 57 est desserrée.

Dans ce mode de réalisation, la vis 56, 57, lorsqu'elle est serrée (figure 9), garantit la sécurité du dispositif, en empêchant tout déplacement accidentel des pièces femelle et mâle 41, 42 l'une par rapport à l'autre. Cette vis 56, 57 pourrait néanmoins, en variante, être supprimée.

On appréciera qu'avec ce dispositif, l'espacement entre les pièces femelle et mâle 41, 42 peut être réglé simplement, en distrayant ces pièces jusqu'à la position souhaitée, et ceci sans qu'un faux mouvement puisse modifier le réglage avant le serrage de la vis 56, 57 en comprimant les pièces 41, 42. Cet effet est obtenu sans fragiliser la pièce mâle 42, qui n'a pas besoin d'être élastique, et sans nécessiter de tolérances de fabrication très contraignantes en ce qui concerne le diamètre externe de la pièce mâle 42 et le diamètre interne de la pièce femelle 41.

Les figures 11 à 16 montrent un autre dispositif de remplacement vertébral 60. Le dispositif 60 comprend des première et seconde pièces allongées, creuses et imbriquées l'une dans l'autre 61, 62.

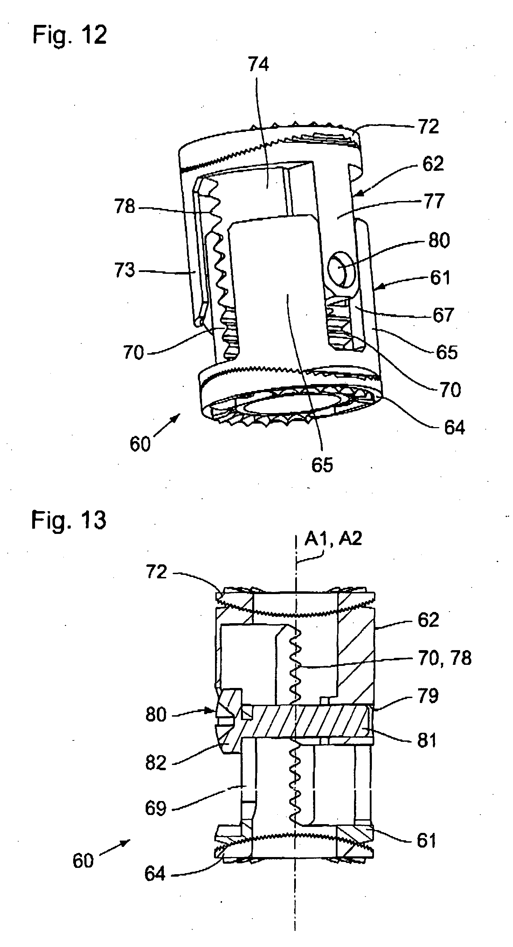

La première pièce ou « pièce inférieure » 61 comprend (cf. figures 11-14) une base circulaire annulaire 63 dont la face inférieure, crantée, coopère avec un plateau d'inclinaison réglable 64 identique au plateau 7 du dispositif 1 et dont la face supérieure porte deux paires de parois en arc de cercle, respectivement 65 et 66, s'étendant dans la direction axiale de la pièce 61. Les deux parois en arc de cercle 65 se situent sur le diamètre extérieur de la base 63 et sont séparées par une fente longitudinale 67 ouverte en partie supérieure (cf. figure 12). Les deux parois 66 se situent sur un diamètre inférieur au diamètre extérieur de la base 63 et sont réunies par une paroi plane 68 comportant une lumière oblongue longitudinale 69 située en regard de la fente 67. Le flanc de chacune des parois 66 éloigné de la paroi plane 68 comporte un crantage en dents de scie 70.

La seconde pièce ou « pièce supérieure » 62 comprend (cf. figures 11-13 et 15) une base circulaire annulaire 71 dont la face supérieure, crantée, coopère avec un plateau d'inclinaison réglable 72 identique au plateau 8 du dispositif 1 et de la face inférieure de laquelle font saillie longitudinalement une paroi en arc de cercle 73 située sur le diamètre extérieur de la base 71 et une paire de parois en arc de cercle 74 située sur un diamètre inférieur au diamètre extérieur de la base 71. Les parois 74 sont réunies par une paroi plane 75 située en regard d'une échancrure 76 pratiquée dans la partie inférieure de la paroi 73. Une nervure longitudinale 77 fait saillie radialement vers l'extérieur depuis la paroi plane 75 jusqu'au diamètre extérieur de l'embase 71. Le flanc de chacune des parois en arc de cercle 74 éloigné de la paroi plane 75 comporte un crantage en dents de scie 78 destiné à coopérer avec le crantage 70 des parois 66 de la pièce inférieure 61 pour définir la position axiale des pièces inférieure et supérieure 61, 62 l'une par rapport à l'autre. La nervure 77 et la paroi plane 75 présentent un alésage taraudé 79.

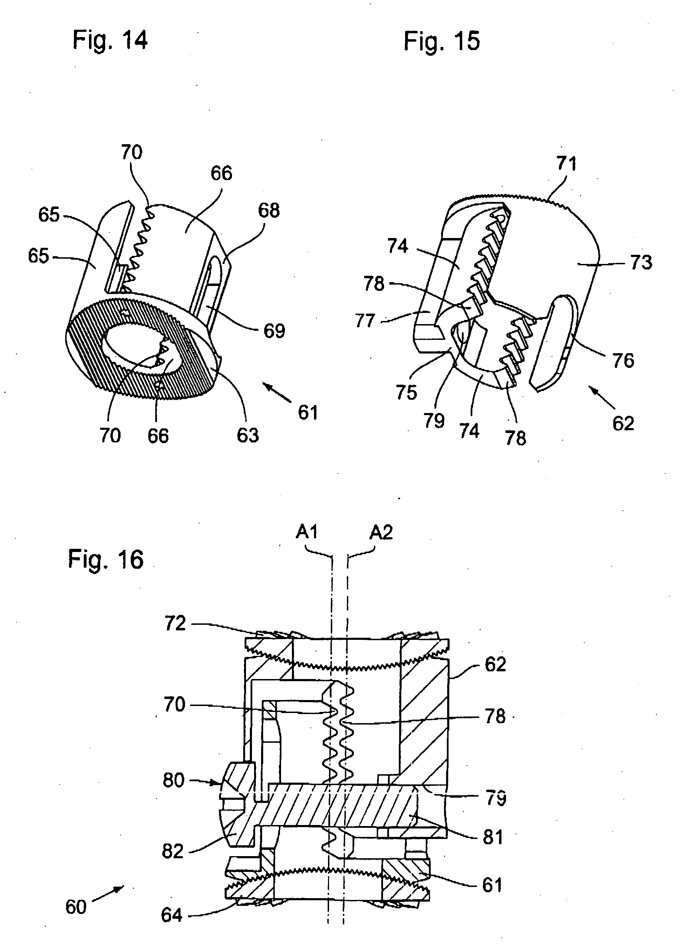

Le dispositif 60 comprend en outre une vis de commande radiale 80 (cf. figures 11-13) passant à travers la lumière oblongue 69 de la pièce inférieure 61, vis 80 dont l'extrémité 81, filetée, coopère avec l'alésage taraudé 79 de la pièce supérieure 62 et dont la tête 82 s'appuie contre la face extérieure de la paroi plane 68. Une rotation de la vis 80 autour de son axe permet de commander un déplacement radial des pièces inférieure et supérieure 61, 62 l'une par rapport à l'autre, entre une position radiale d'ancrage et une position radiale écartée. Dans la position radiale d'ancrage (figures 11-13), correspondant à une position serrée de la vis 80, les axes longitudinaux A1, A2 des pièces 61, 62 sont confondus et les crantages 78 de la pièce supérieure 62 coopèrent avec les crantages 70 de la pièce inférieure 61 et définissent ainsi une position axiale relative des pièces 61, 62. Dans la position radiale écartée (figure 16), correspondant à une position desserrée de la vis 80, les axes A1, A2 des pièces 61, 62 sont disjoints, les crantages 78 de la pièce supérieure 62 sont éloignés de ceux, 70, de la pièce inférieure 61 et les pièces inférieure et supérieure 61, 62 sont libres de coulisser axialement l'une par rapport à l'autre, permettant le réglage de l'espacement axial entre ces pièces 61, 62. Les déplacements radial et axial des pièces 61, 62 l'une par rapport à l'autre sont guidés par la nervure 77 qui coopère avec la fente 67

(figure 12) et par la tige de la vis 80 qui coopère avec la lumière oblongue 69. Le déplacement axial est limité par la lumière oblongue 69 contre la paroi de laquelle peut venir buter la tige de la vis 80. Le déplacement radial est lui limité par les parois 65, 66 de la pièce inférieure 61 contre lesquelles peuvent venir buter respectivement les parois 74 et la paroi 73 de la pièce supérieure 62.

On notera que dans la position radiale d'ancrage des pièces 61, 62, l'ancrage s'effectue de part et d'autre de l'axe A1, A2 du dispositif 60, de par la position des crantages 70, 78. Dans cette position, la vis 80 bloque complètement et de manière fiable les pièces 61, 62 l'une par rapport à l'autre.

## Revendications

1. Dispositif de remplacement vertébral (1) comprenant une pièce femelle (2), une pièce mâle (3) montée dans la pièce femelle (2) et pouvant être déplacée axialement par rapport à la pièce femelle (2), et des moyens pour bloquer les pièces femelle et mâle (2, 3) axialement l'une par rapport à l'autre, **caractérisé en ce que** les moyens de blocage comprennent :
- des premier et second éléments de blocage (14,15) montés mobiles en translation radiale sur la pièce femelle (2) pour occuper une première position dans laquelle ils coopèrent avec des parties respectives opposées (31) d'une surface périphérique (6) de la pièce mâle (3) pour bloquer les pièces femelle et mâle (2, 3) axialement l'une par rapport à l'autre et une seconde position dans laquelle les pièces femelle et mâle (2, 3) peuvent être déplacées axialement librement l'une par rapport à l'autre, et
- un organe rotatif de commande (20) s'étendant radialement à travers la pièce mâle (3), cet organe de commande (20) coopérant avec les éléments de blocage (14, 15) pour, lorsqu'un mouvement de rotation lui est imprimé, transformer ce mouvement de rotation en un mouvement de translation de chacun des éléments de blocage (14, 15) pour amener les éléments de blocage (14, 15) dans l'une ou l'autre des première et seconde positions.

2. Dispositif selon la revendication 1, **caractérisé en ce que** les éléments de blocage (14, 15) sont guidés dans des logements respectifs (12, 13) pratiqués dans une paroi périphérique (11) de ia pièce femelle (2).

3. Dispositif selon la revendication 1 ou 2, **caractérisé en ce que** les éléments de blocage (14, 15) comportent chacun un crantage (30) coopérant avec un crantage complémentaire (31) de la surface périphérique (6) de la pièce mâle (3) lorsque les éléments de blocage (14, 15) sont dans la première position.

4. Dispositif selon l'une quelconque des revendications 1 à 3, **caractérisé en ce qu'**au moins le second élément de blocage (15) est bloqué en rotation par rapport à la pièce femelle (2), **en ce que** l'organe de commande (20) est une vis comprenant une tête (21) et une tige (22) dont l'extrémité la plus éloignée de la tête (21) est filetée et coopère avec un taraudage (17) du second élément de blocage (15), et **en ce que** le premier élément de blocage (14) est monté libre autour d'une partie non filetée de la tige (22) de la vis (20), proche de la tête (21), de manière à être poussé par la tête (21) lorsque la vis est tournée dans un sens tendant à amener les éléments de blocage (14, 15) dans leur première position.

5. Dispositif selon la revendication 4, **caractérisé en ce que** le premier élément de blocage (14) est bloqué en rotation par rapport à la pièce femelle (2).

6. Dispositif selon la revendication 4 ou 5, **caractérisé en ce que** les éléments de blocage (14, 15) sont soumis à une action élastique qui tend à écarter chacun des éléments de blocage (14, 15) de la pièce mâle (3) et à maintenir le premier élément de blocage (14) en appui contre la tête (21) de la vis (20).

7. Dispositif selon la revendication 6, **caractérisé en ce que** ladite action élastique est exercée par des première et seconde lames ressort (23, 24) agissant respectivement sur les premier et second éléments de blocage (14, 15), ces lames ressort (23, 24) faisant partie d'une même pièce monobloc (25).

8. Dispositif selon l'une quelconque des revendications 1 à 7, **caractérisé en ce que** l'organe de commande (20) comprend des moyens (32) de réception d'un instrument de commande en rotation.

9. Dispositif selon l'une quelconque des revendications 1 à 8, **caractérisé en ce que** la pièce mâle (3) est creuse et comporte dans sa paroi périphérique des première et seconde lumières (18, 19) oblongues opposées et parallèles à l'axe (A) de la pièce mâle (3) pour le passage de l'organe de commande (20).

10. Dispositif selon l'une quelconque des revendications 1 à 9, **caractérisé en ce qu'**au moins l'une desdites pièces (2, 3) est fixée à son extrémité la plus éloignée de l'autre desdites pièces à un plateau d'inclinaison réglable (7, 8), ce ou chacun de ces plateaux (7, 8) comprenant une face crantée (35) ayant un profil en arc de cercle et coopérant avec une face crantée (36) de forme complémentaire de la pièce correspondante (2, 3) pour définir l'inclinaison dudit plateau.

11. Dispositif selon la revendication 10, **caractérisé en ce que** le ou chacun des plateaux d'inclinaison réglable (7, 8) est fixé à la pièce (2, 3) correspondante au moyen d'au moins une vis (9) coopérant avec une ouverture oblongue correspondante (34) du plateau, cette ou ces lumières oblongues (34) étant orientées orthogonalement à une direction dans laquelle s'étend le crantage (35) du plateau.

## Claims

1. Vertebral replacement device (1) comprising a female part (2), a male part (3) that is mounted in the female part (2) and is displaceable axially relative to the female part (2), and means for axially locking the female and male parts (2, 3) to one another, **characterised in that** the locking means comprises:
- first and second locking elements (14, 15) mounted so as to be movable in radial translation on the female part (2), in order to occupy a first position at which they cooperate with respective opposing parts (31) of a peripheral surface (6) of the male part to axially lock the female and male parts (2, 3) to one another, and a second position at which the female and male parts (2, 3) are freely displaceable axially relative to one another, and
- a rotating control element (20) extending radially through the male part (3), said control element (20) cooperating with the locking elements (14, 15) so that when a rotational movement is communicated to the control element, said rotational movement is transformed into a translational movement of each of the locking elements (14, 15) so as to bring the locking elements (14, 15) into either of the first or second positions.

2. Device according to claim 1, **characterised in that** the locking elements (14, 15) are guided in respective recesses (12, 13) provided in a peripheral wall (11) of the female part (2).

3. Device according to claim 1 or 2, **characterised in that** the locking elements (14, 15) each comprise a notching (30) that cooperates with a complementary notching (31) of the peripheral surface (6) of the male part (3) when the locking elements (14, 15) are in the first position.

4. Device according to any one of claims 1 to 3, **characterised in that** at least the second locking element (15) is rotationally fixed relative to the female part (2), **in that** the control element (20) is a screw comprising a head (21) and a shaft (22) whose end furthest from the head (21) is threaded and cooperates with a tapping (17) of the second locking element (15), and **in that** the first locking element (14) is freely mounted around an unthreaded part of the shaft (22) of the screw (20) near the head (21), so as to be pushed by the head (21) when the screw is turned in a direction that tends to bring the locking elements (14, 15) into their first position.

5. Device according to claim 4, **characterised in that** the first locking element (14) is rotationally fixed relative to the female part (2).

6. Device according to claim 4 or 5, **characterised in that** the locking elements (14, 15) are subjected to an elastic action that tends to move each of the locking elements (14, 15) away from the male part (3) and to keep the first locking element (14) pressed against the head (21) of the screw (20).

7. Device according to claim 6, **characterised in that** said elastic action is exerted by first and second spring leaves (23, 24), respectively acting on the first and second locking elements (14, 15), these spring leaves (23, 24) forming part of a same unitary part (25).

8. Device according to any one of claims 1 to 7, **characterised in that** the control element (20) comprises means (32) for receiving a rotational driving instrument.

9. Device according to any one of claims 1 to 8, **characterised in that** the male part (3) is hollow and comprises in its peripheral wall first and second opposing oblong holes (18, 19) extending parallel to the axis (A) of the male part (3) for the passage of the control element (20).

10. Device according to any one of claims 1 to 9, **characterised in that** at least one of said parts (2, 3) is attached at its end furthest from the other of said parts to an adjustably inclinable plate (7, 8), said plate or each of these plates (7, 8) comprising a notched surface (35) having an arc-shaped profile and cooperating with a notched surface (36) of complementary shape of the corresponding part (2, 3) to define the inclination of said plate.

11. Device according to claim 10, **characterised in that** the or each adjustably inclinable plate (7, 8) is attached to the corresponding part (2, 3) by means of at least one screw (9) cooperating with a corresponding oblong hole (34) of the plate, said oblong hole(s) (34) being oriented orthogonal to a direction in which the notching (35) of the plate runs.

## Patentansprüche

1. Wirbelersatzvorrichtung (1), umfassend einen Aufnahmeteils (2), einen in dem Aufnahmeteil (2) befestigten Einsteckteil (3), der axial in Bezug zum Aufnahmeteil (2) verschoben werden kann, und Mittel, um den Einsteck- und den Aufnahmeteil (2, 3) axial zueinander festzustellen, **dadurch gekennzeichnet, dass** die Feststellungsmittel umfassen:
- erste und zweite Feststellungsmittel (14, 15), die in radialer Translation auf dem Aufnahmeteil (2) beweglich montiert sind, um eine erste Position, in der sie mit jeweils gegenüber liegenden Teilen (31) einer Umfangsfläche (6) des Einsteckteils (3) zusammenwirken, um den Aufnahme- und den Einsteckteil (2, 3) axial zueinander festzustellen, und eine zweite Position einzunehmen, in der der Aufnahme- und der Einsteckteil (2, 3) axial frei zueinander verschoben werden können, und
- ein drehbares Steuerelement (20), das sich radial durch den Einsteckteil (3) erstreckt, wobei dieses Steuerelement (20) mit den Feststellelementen (14, 15) zusammenwirkt, um, wenn es einer Drehbewegung unterzogen wird, diese Drehbewegung in eine Translationsbewegung jedes der Feststellelemente (14, 15) umzuwandeln, um die Feststellelemente (14, 15) in die eine oder die andere der ersten und zweiten Positionen zu bringen.

2. Vorrichtung nach Anspruch 1, **dadurch gekennzeichnet, dass** die Feststellelemente (14, 15) in jeweiligen Lagerungen (12, 13) geführt werden, die in einer Umfangswand (11) des Aufnahmeteils (2) vorgesehen sind.

3. Vorrichtung nach Anspruch 1 oder 2, **dadurch gekennzeichnet, dass** die Feststellelemente (14, 15) jeweils eine Zahnung (30) umfassen, die mit einer komplementären Zahnung (31) der Umfangsfläche (6) des Einsteckteils (3) zusammenwirkt, wenn die Feststellelemente (14, 15) in der ersten Position sind.

4. Vorrichtung nach einem der Ansprüche 1 bis 3, **dadurch gekennzeichnet, dass** mindestens das zweite Feststellelement (15) in Drehung in Bezug zum Aufnahmeteil (2) festgestellt wird, dass das Steuerelement (20) eine Schraube mit einem Kopf (21) und einem Schaft (22) ist, dessen am weitesten vom Kopf (21) entferntes Ende ein Gewinde besitzt und mit einem Gewinde (17) des zweiten Feststellelements (15) zusammenwirkt, und dass das erste Feststellelement (14) frei um einen Teil des Schafts (22) der Schraube (20) ohne Gewinde nahe dem Kopf (21) montiert ist, um vom Kopf (21) geschoben zu werden, wenn die Schraube in eine Richtung gedreht wird, die dazu neigt, die Feststellelemente (14, 15) in ihre erste Position zu bringen.

5. Vorrichtung nach Anspruch 4, **dadurch gekennzeichnet, dass** das erste Feststellelement (14) in Drehung in Bezug zum Aufnahmeteil (2) festgestellt wird.

6. Vorrichtung nach Anspruch 4 oder 5, **dadurch gekennzeichnet, dass** die Feststellelemente (14, 15) einer elastischen Betätigung unterzogen werden, die dazu neigt, jedes der Feststellelemente (14, 15) von dem Einsteckteil (3) zu entfernen und das erste Feststellelement (14) am Kopf (21) der Schraub (20) anliegend zu halten.

7. Vorrichtung nach Anspruch 6, **dadurch gekennzeichnet, dass** die elastische Betätigung durch erste und zweite Federblätter (23, 24) ausgeübt wird, die auf die ersten bzw. zweiten Feststellelemente (14, 15) einwirken, wobei diese Federblätter (23, 24) Teil eines selben Stückes (25) sind.

8. Vorrichtung nach einem der Ansprüche 1 bis 7, **dadurch gekennzeichnet, dass** das Steuerelement (20) Mittel (32) für die Aufnahme eines drehbaren Steuerinstruments umfasst.

9. Vorrichtung nach einem der Ansprüche 1 bis 8, **dadurch gekennzeichnet, dass** der Einsteckteil (3) hohl ist und in seiner Umfangswand erste und zweite gegenüber liegende längliche Öffnungen (18, 19) parallel zur Achse (A) des Einsteckteils (3) für den Durchgang des Steuerelements (20) umfasst.

10. Vorrichtung nach einem der Ansprüche 1 bis 9, **dadurch gekennzeichnet, dass** mindestens einer der Teile (2, 3) an seinem am weitesten von dem anderen der Teile entfernten Ende an einer Platte (7, 8) mit einstellbarer Neigung befestigt ist, wobei diese oder jede dieser Platten (7, 8) eine gezahnte Fläche (35) mit einem Kreisbogenprofil umfasst, die mit einer gezahnten Fläche (36) von komplementärer Form des entsprechenden Teils (2, 3) zusammenwirkt, um die Neigung der Platte zu definieren.

11. Vorrichtung nach Anspruch 10, **dadurch gekennzeichnet, dass** die oder jede der Platten (7, 8) mit einstellbarer Neigung an dem entsprechenden Teil (2, 3) mit Hilfe mindestens einer Schraube (9) befestigt ist, die mit einer entsprechenden länglichen Öffnung (34) der Platte zusammenwirkt, wobei diese längliche(n) Öffnung(en) (34) orthogonal zu einer Richtung ausgerichtet ist (sind), in die sich die Zahnung (35) der Platte erstreckt.
